# EUROPEAN PATENT SPECIFICATION

(11) **EP 0 963 745 B1**
(45) Date of publication and mention of the grant of the patent: **19.02.2003**
(21) Application number: 99304441.1
(22) Date of filing: 08.06.1999
(51) Int. Cl.: A61F 13/02, A61F 13/00, A61F 13/15, B32B 5/26, D04H 13/00

(54) **Non-woven absorbent materials having tabs**
Absorbierende Vliesstoffmaterialien mit Klebebändern
Matériaux absorbants non-tissés ayant des languettes

(30) Priority: 13.06.1998 GB 9812692
(43) Date of publication of application: 15.12.1999
(73) Proprietor: Lohmann GmbH & Co. KG, 56567 Neuwied (DE)
(72) Inventor: Barth, Georg Martin, 56579 Rengsdorf (DE); Carus, Edmund Hugh, Clitheroe, Lancashire, BB7 9QJ (GB)
(74) Representative: Barker, Rosemary Anne

(56) References cited:
- EP-A- 0 531 096
- EP-A- 0 747 027
- DE-A- 1 492 362
- US-A- 3 900 027

## Description

The present invention relates to non-woven textile materials and in particular to non-woven absorbent textile materials.

The present invention has particular application to non-woven absorbent textile materials for uses in a medical environment, for example, dressings, and swabs for use in operating theatres.

Non-woven absorbent textile materials have been used as a substitute for woven materials for a considerable number of years.

Modern manufacturing techniques, and in particular hydroentanglement, are able to reproduce the properties of a woven textile material whilst in addition allowing for the elimination of problems which arise in woven materials.

Non-woven materials allow for easier conversion since they do not possess the problems of fraying selvedges and consequent complex folding complications to tuck-in this selvedge.

Furthermore, the use of non-woven materials enables the provision of enhanced absorbency and hence the use of fewer material layers. Also loose fibres on the surface of the material are substantially eliminated.

EP-A-0 531 096 (McNeil-PPC Inc.) discloses an absorbent composite fabric for use as a wound dressing. One version is composed of an absorbent non-woven fibrous layer sandwiched between respective layers of lightweight, water-permeable hydrophobic fibrous material, the layers being bonded by hydraulic entanglement.

The present invention seeks to provide an improved non-woven absorbent material which includes means for handling the material when formed into a product e.g. a swab, and which can also include radio-opaque materials

The present invention also seeks to provide means for manufacturing the above non-woven absorbent material.

Accordingly, the present invention provides a non-woven absorbent material comprising a core of absorbent fibrous material and respective outer layers of hydrophobic or semi-hydrophobic fibrous material formed into a composite by web consolidation, characterised in that a series of tapes or polymer strips are incorporated in spaced apart relationship in order to provide integral tabs or flaps when the material is cut.

Such tabs or flaps are useful for improved hygiene when handling or positioning the finished products, which may comprise wound dressings, and swabs and pads for medical and other sanitary purposes. In particular, products incorporating such tabs can be positioned exactly and correctly in surgical and other medical procedures thus providing significant advantages over existing swab and related products.

The tapes or strips which provide the integral tabs or flaps are preferably bonded longitudinally to one of the outer layers of the composite. In this respect, they are preferably compressed into the surface of the composite so that the latter has its surface correspondingly formed into a series of longitudinal ridges and recesses. Where the tabs are formed from polymer-based strips, they may be heat bonded to the composite. Where the tabs are formed of tapes of fibrous material, heat bonding may also be possible if the material is impregnated with a thermosetting substance. In either case, adhesive may alternatively be used for bonding.

The tapes or strips can be made radio-opaque, for example, by incorporating barium sulphate.

In preferred embodiments in accordance with the invention, the respective outer layers of the composite have a minimum weight of 60g/m² and are formed of fibres which are at least 6 denier in diameter.

In this respect, it is preferable that the fibres of the outer layers are of a certain weight (60g/m² or more) and size (6 denier or more) in order that the final material will drape and handle in a suitable manner for use as a medical dressing, and also in order that the surface of the material is substantially non-adherent, a property also very important in the case of a wound dressing. These fibres also provide spaces to hold and store absorbed fluids due to the large interfibre spaces attained with the weight and dimensions described.

The fibres of the outer layers are also preferably of a relatively long staple length e.g. greater than 60mm.

These fibres can comprise polypropylene including polypropylene made utilising metallocene technology, polyester and polyamide or any other suitable hydrophobic fibre or semi-hydrophobic fibre or blends of these fibres.

The outer layers can also include a small quantity of hydrophilic fibres in order to aid the transport of fluid or exudate to the inner core.

Alternatively, hydrophobic fibres with a hydrophilic finish can be used, all or in part, to assist fluid management.

The core comprises hydrophilic fibres, for example, of regenerated rayon or solvent spun rayons. Other hydrophilic fibres known to those skilled in the art are equally appropriate, all or in part.

Preferably, the fibres of the core are relatively fine in diameter e.g. 3 denier or less and are of a relatively short staple length e.g. 38mm or less.

Each of the layers of the non-woven absorbent material can be prepared by standard or specialist web forming techniques which are used in staple fibre non-woven manufacture.

The webs forming the layers of the composite material are preferably subjected to a hydroentangling process in order to produce a single layer integrated finished composite non-woven material, which due to the hydroentangling process will exhibit no propensity to delaminate into the precursor webs. Such a process has no need for chemical binders which are required in production of non-woven textile products and have to be removed from sterile products such as dressings and swabs.

However, the composite material of the invention, with the integrally bonded tapes or strips could also be formed by suitable alternative methods, such as needle punching, or thermal bonding, or ultrasonic bondings - all known methods of bonding together layers of fibrous materials.

A material according to the present invention can have very similar handling properties to woven textile material but also has an increased absorbency and increased resistance to surface abrasion compared to a similar woven textile based equivalent. Such material will exhibit minimal linting or fibre shedding in use, particularly when manipulated by forceps, or the like, in the end use.

The use of the hydroentanglement process enables the incorporation of handling and/or application instructions on the finished dressings or swabs by imprinting and/or embossing and/or moulding using an appropriate screen design in the hydroentanglement process. Also, this process can impart abrasive properties on the side printed.

The fibres of the inner core may advantageously include fibres which swell rapidly upon absorption of water, such as cellulose derivatives such as cross-linked carboxymethyl cellulose, or other comparable materials. These fibres will swell during the hydroentanglement process, as water is sprayed onto the web and should therefore increase the thickness/bulk of the finished material notwithstanding the drying stage which follows the hydroentanglement process. This intentional swelling and subsequent fibre collapse in the drying stage provides absorbency and protection against disintegration in use.

A material according to the present invention can also include thermoplastic or other bonding matrices such as netting or powder bonding materials or relevant fibres in order to provide optimised strength characteristics. These ingredients must withstand relevant sterilisation procedures.

These materials can be activated by heat using thermofusion, which is particularly appropriate to fibres and netting, or ultrasonics or infra-red techniques.

A material according to the present invention can also include bactericidal fibres or other materials which are effective against virulent bacteria, for example MRSA.

Radio-opaque materials or members can be included in the material according to the present invention either in the form of separate threads, monofilaments, tapes or printed formats, or, as previously mentioned, as additions to the tapes or polymer strips which are incorporated into the present material to provide tabs or flaps for end uses where positioning is essential.

For example, radio-opaque threads can be deposited in a spaced-apart relationship (dependent on the width of the material produced) to ensure that every swab or dressing contains such a radio-opaque member on conversion) between the core web and one of the outer webs during the manufacturing process.

An oblique or zig-zag course of deposition of the radio-opaque thread in the composite is preferred. Since the radio-opaque thread can be coarser in diameter than the other fibres used in the composite this avoids formation of a ridge and potential breakage when the finished material is stored as a roll. If no deposition control is adopted, localised build-up where the radio-opaque member is deposited will occur. This can result in high stresses in the radio-opaque material and its resultant rupture or severe weakening as the roll of composite gets larger in diameter.

The radio-opaque threads, tapes or other formats ideally comprise a polypropylene or polyester based matrix which can include up to 80% by weight barium sulphate in order to provide maximum traceability under X-rays. Coupling agent technology using, for example, organo-titanates, facilitates this high incorporation of barium sulphate whilst maintaining the physical properties of the matrix polymer. A preferred matrix polymer is polypropylene made using metallocene technology which exhibits improved resistance to gamma sterilization over the regular types used commonly to date.

The composite material according to the present invention can include environmental and/or infection control options utilizing dry-spun polyvinyl alcohol derivatives, sulphonated polyesters, water soluble cellulose acetate polymers or other technologies known to be effective in infection control. Such materials can be used, all or in part as a substitute for the polymers cited for the hydrophobic and hydrophilic components of the composite according to the present invention.

The absorbency of the material can be enhanced by utilizing highly absorbent fibres, microfibres including bacterially generated cellulose, fibrillatable fibres particularly suitable for high pressure hydroentanglement, shaped fibres or blends of all or some of these fibres in the core layer or layers. It is possible to optimise absorbency and reduce composite bulk by these technologies.

The integrated single layer composite allows for the production and in-line converting of swabs, a significant advantage over any conversion procedures to date used with either traditional woven gauze or non-woven gauze. Significant hygiene and product cleanliness is possible since the manufacturing and the extremely simplified converting required can be totally encased in a controlled environment with minimal exposure to the natural climate and manual handling. Contamination risks are hence dramatically reduced in the composite according to the present invention.

In addition to the reduced contamination risks with the production of the composite according to the present invention, the elimination of folding provides significant cost savings.

Furthermore, shaped swabs become readily possible to provide for better in-use performance.

The present invention will now be more particularly described with reference to the accompanying drawings in which:
Figure 1 is a diagrammatic representation of the manufacture of one form of a non-woven absorbent material according to the present invention;
Figure 2a is a diagrammatic elevation of a non-woven absorbent material according to the present invention;
Figure 2b is a plan view of the material shown in Figure 2a;
Figure 3 is a diagrammatic plan view of the manufacture of dressings or swabs including handling means, according to an embodiment of the present invention;
Figure 4 is a diagrammatic side view of the material shown in Figure 3;
Figure 5 is a diagrammatic sketch showing how the material of Figures 3 and 4 is bonded to the tapes and cut;
Figure 6 illustrates diagrammatically a completed dressing or swab formed from the material shown in figures 3 and 4;
Figure 7 illustrates diagrammatically another embodiment of the invention whereby the radio-opaque element in multi-filament form is introduced between the fibrous layers adjacent to the middle layer; and
Figure 8 illustrates diagrammatically a completed dressing or swab formed from the material shown in Figure 7.

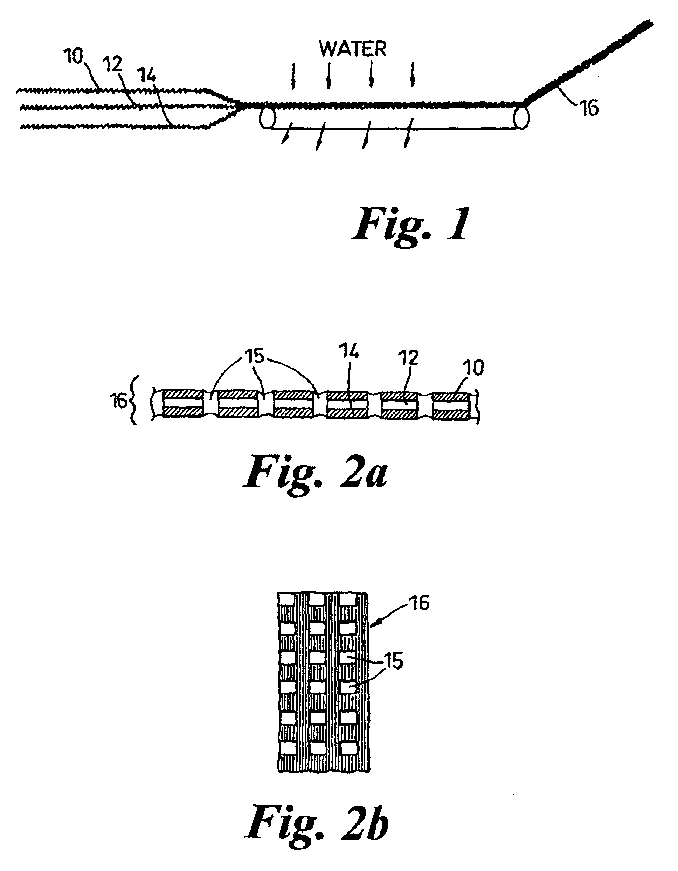

Referring to Figure 1, there is shown three webs of fibrous material (10, 12, 14), the outer webs (10, 14) being formed from hydrophobic or semi-hydrophobic fibres having a minimum weight of 60g/m² and a minimum diameter of 6 denier, whilst the central web (12) is formed from hydrophilic fibres. The webs (10, 12, 14) are free of binders or any other chemical additives.

The webs (10, 12, 14) are bonded or consolidated together by a hydroentanglement process to produce an integrated composite material (16). In this respect, high pressure jets of water are fixed at respective sides of the combined web, the water passing through and being recirculated after filtration.

The degree of hydroentanglement can be controlled by use of appropriate screens from open structure e.g. 100 apertures/square cm to very finely holed assemblies where the apertures are barely visible.

The outer layers (10, 14) are either wholly non-absorbent in nature or have a small degree of absorbency which is provided by the inclusion of, for example, a proportion of hydrophilic fibres in order to assist the transport of fluid to the central layer (12) which is highly absorbent in nature. Figures 2a and 2b illustrate a coarse apertured version of the integrated composite (16) according to the present invention, the apertures being designated by reference (15).

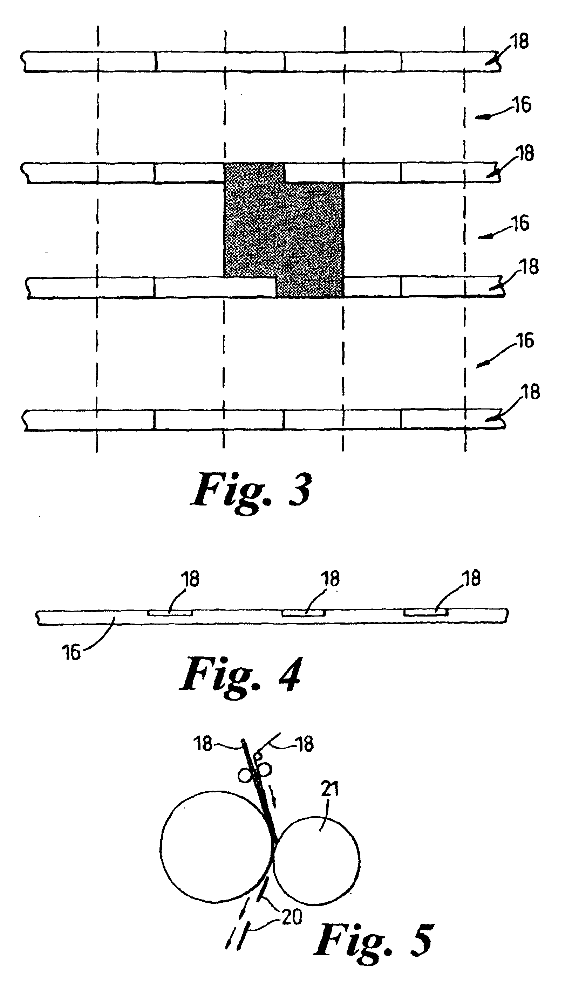

Referring to Figures 3, 4 and 5, a series of tapes (18), which may contain a high proportion by weight of barium sulphate and therefore be radio opaque, are introduced during the manufacturing process.

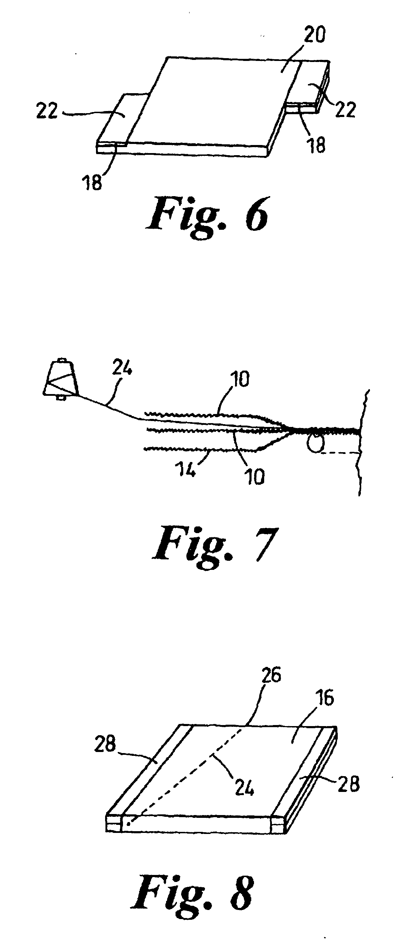

The tapes (18) are laid on top of one side of the composite (16). They are then bonded to the composite by heat or other means, and also compressed into the surface thereof. Subsequent to this bonding process, a rotary cutter (21), or similar means are used to cut or slit the composite web to produce the finished dressing or swab (20), of a shape corresponding to the shaded region in Figure 3. The completed dressing or swab (20) is also illustrated in Figure 6. The tape (18), which may be of radio-opaque material, forms integral tabs or flaps (22) which allow for easy and accurate positioning of the dressing or swab in use, whilst optionally also imparting radio-opacity.

In Figure 7, another embodiment of the invention is illustrated diagrammatically whereby a radio-opaque member in multi-filament form (24) is introduced between the fibrous layers (10, 14) adjacent to the middle layer (12) prior to hydroentanglement to secure said member firmly into the integrated composite. As shown in Figure 8, which illustrates a completed dressing or swab (26), the radio-opaque member extends obliquely therethrough to prevent potential for breakage when formed into a roll. In this embodiment, integral flaps (28) at each side of the dressing (26) extend the full length of the product but are only half the original width of the integrated tape (18), the latter having been cut (or slit) along a midline, instead of in the stepped manner shown in Figure 3.

These illustrations are for example only.

## Claims

1. A non-woven absorbent material comprising a core of absorbent fibrous material and respective outer layers of hydrophobic or semi-hydrophobic fibrous material formed into a composite material by web consolidation, **characterised in that** a series of tapes or polymer strips are incorporated in spaced apart relationship in order to provide integral tabs or flaps when the material is cut.

2. A material as claimed in claim 1 in which the tapes or strips are bonded longitudinally to one of the outer layers.

3. A material as claimed in claim 2 in which the tapes or strips are compressed into the surface of the one outer layer.

4. A material as claimed in claim 1, 2 or 3 in which the tapes or strips consist of or include adhesive material.

5. A material as claimed in claim 1, 2 or 3 in which the tapes or strips consist of or include radio opaque material.

6. A material as claimed in any one of claims 1 to 4 in which radio-opaque materials or members in the form of threads, monofilaments or printed formats are incorporated in the core and/or at least one of the outer layers.

7. A material as claimed in claim 6 in which the radio-opaque materials or members extend obliquely or in a zig zag course through the core and/or at least one of the outer layers.

8. A material as claimed in any one of the preceding claims in which the respective outer layers have a minimum weight of 60g/m² and are formed of fibres which are at least 6 denier in diameter.

9. A material as claimed in any one of the preceding claims in which the fibres of the outer layers are of a relatively long stable length e.g. greater than 60mm.

10. A method of forming a material as claimed in any one of the preceding claims in which a central web of absorbent fibrous material and two outer webs of hydrophobic or semi-hydrophobic fibrous material are subjected to a hydroentangling process in order to produce a single layer integrated finish composite non-woven material, in which a series of tapes are incorporated in a spaced apart relationship, and in which the material is subsequently cut to provide individual products having sections of the tapes as integral tabs or flaps.

## Revendications

1. Matériau absorbant non tissé comprenant un noyau de matériau fibreux-absorbant et des couches externes respectives de matériau fibreux hydrophobe ou semi hydrophobe formé en un matériau composite par consolidation de couche, **caractérisé en ce que** plusieurs rubans ou bandes de polymère sont incorporés en relation espacée afin de fournir des lames ou volets monoblocs lorsque le matériau est coupé.

2. Matériau selon la revendication 1, dans lequel les rubans ou bandes sont liés (ées) longitudinalement à l'une des couches externes.

3. Matériau selon la revendication 2, dans lequel les rubans ou bandes sont comprimés (ées) dans la surface de l'un des couches externes.

4. Matériau selon la revendication 1, 2 ou 3, dans lequel les rubans ou bandes sont constitués (ées) ou comprennent du matériau adhésif.

5. Matériau selon la revendication 1, 2 ou 3, dans lequel les rubans ou bandes sont constitués (ées) ou comprennent un matériau impénétrable aux radiations.

6. Matériau selon l'une quelconque des revendications 1 à 4, dans lequel des matériaux ou éléments impénétrables aux radiations sous la forme de fils, mono-filaments, ou formats imprimés sont incorporés dans le noyau et/ou au moins une des couches externes.

7. Matériau selon la revendication 6, dans lequel les matériaux ou éléments impénétrables aux radiations s'étendent de manière oblique ou en zig-zag à travers le noyau et/ou au moins une des couches externes.

8. Matériau selon l'une quelconque des revendications précédentes, dans lequel les couches externes respectives ont un poids minimum de 60g/m² et sont formées de fibres qui ont au moins 6 deniers en diamètre.

9. Matériau selon l'une quelconque des revendications précédentes, dans lequel les fibres des couches externe ont une longueur stable relativement longue, par exemple supérieure à 60 mm.

10. Procédé pour former un matériau selon l'une quelconque des revendications précédente, dans lequel une couche centrale de matériau fibreux-absorbant et deux couches externes en matériau fibreux hydrophobe ou semi hydrophobe sont soumises à un processus d'hydroemmêlage de fils pour produire un matériau non tissé composite fini monobloc mono-couche, dans lequel plusieurs rubans sont incorporés dans une relation espacée, et dans lequel le matériau est ensuite coupé pour fournir des produits individuels ayant des sections des rubans en tant que lames ou volets monoblocs.

## Patentansprüche

1. Absorbierendes nicht gewebtes Material beinhaltend einen Innenteil aus absorbierendem faserigen Material und zugehörige äußere Schichten aus wasserabweisendem oder teilweise wasserabweisendem faserigen Material, die durch Bahnverfestigung in ein Verbundmaterial umgeformt werden, **dadurch gekennzeichnet, dass** eine Anzahl von Bändern oder Polymer-Streifen im Abstand zueinander eingelagert sind, um integrierte Laschen oder Lappen bereitzustellen, wenn das Material zerschnitten wird.

2. Material nach Anspruch 1, wobei die Bänder oder Streifen der Länge nach mit einer der äußeren Schichten verbunden sind.

3. Material nach Anspruch 2, wobei die Bänder oder Streifen in die Oberfläche einer der äußeren Schichten hineingepresst sind.

4. Material nach Anspruch 1, 2 oder 3, wobei die Bänder oder Streifen aus klebendem Material bestehen oder solches beinhalten.

5. Material nach Anspruch 1, 2 oder 3, wobei die Bänder oder Streifen aus strahlenundurchlässigem Material bestehen oder solches beinhalten.

6. Material nach einem der Ansprüche 1 bis 4, wobei strahlenundurchlässige Materialien oder Elemente in der Form von Fäden, Monofilamenten oder gedruckten Formaten dem Innenteil und/oder mindestens einer der äußeren Schichten eingelagert sind.

7. Material nach Anspruch 6, wobei die strahlenundurchlässigen Materialien oder Elemente sich schräg oder in einem Zick-Zack-Verlauf durch den Innenteil und/oder mindestens eine der äußeren Schichten erstrecken.

8. Material wie in einem der vorangehenden Ansprüche beansprucht, wobei die jeweils äußeren Schichten ein Mindestgewicht von 60 g/m^2 aufweisen und aus Fasern gebildet sind, deren Durchmesser mindestens 6 Denier beträgt.

9. Material wie in einem der vorangehenden Ansprüche beansprucht, wobei die Fasern der äußeren Schichten eine relativ lange beständige Länge beispielsweise von mehr als 60mm aufweisen.

10. Verfahren zum Herstellen eines Materials wie in einem der vorangehenden Ansprüche beansprucht, in welchem eine innere Bahn aus absorbierendem faserigen Material und zwei äußere Bahnen aus wasserabweisendem oder teilweise wasserabweisendem faserigen Material einer wasserverfilzenden Bearbeitung unterzogen werden, um ein einschichtiges integriertes fertiges nicht-gewebtes Verbundmaterial zu erhalten, wobei eine Anzahl von Bändern im Abstand zueinander eingelagert sind und wobei das Material anschließend zerschnitten wird, um einzelne Produkte bereit zu stellen, welche Bereiche der Bänder als integrale Laschen oder Lappen aufweisen.
